(19) Europäisches Patentamt
European Patent Office
Office européen des brevets

(11) Veröffentlichungsnummer: **0 659 434 A1**

# (12) EUROPÄISCHE PATENTANMELDUNG

(21) Anmeldenummer: **94119398.9**

(51) Int. Cl.[6]: **A61K 31/675**, C07F 9/6512

(22) Anmeldetag: **08.12.94**

(30) Priorität: **21.12.93 DE 4343599**
**21.12.93 DE 4343600**

(43) Veröffentlichungstag der Anmeldung:
**28.06.95 Patentblatt 95/26**

(84) Benannte Vertragsstaaten:
**AT BE CH DE DK ES FR GB GR IE IT LI LU MC NL PT SE**

(71) Anmelder: **Troponwerke GmbH & Co. KG**
**Berliner Strasse 156**
**D-51063 Köln (DE)**

(72) Erfinder: **Maurer, Fritz, Dr.**
**Nakakuki 5-13-9**
**Oyama-shi,**
**Tochigi 323 (JP)**
Erfinder: **Schmidt, Bernard, Dr.**
**In der Fuhr 8**
**D-51789 Lindlar (DE)**

(74) Vertreter: **Dänner, Klaus, Dr. et al**
**Bayer AG**
**Konzernverwaltung RP**
**Patente Konzern**
**D-51368 Leverkusen (DE)**

## (54) Verwendung von Phosphor- und Phosphonsäureestern zur Behandlung von Hirnleistungsstörungen und Depressionen.

(57) Die vorliegende Erfindung betrifft die Verwendung von Phosphor- und Phosphonsäureestern zur Herstellung von Arzneimitteln zur Behandlung von Hirnleistungsstörungen und affektiven Störungen, insbesondere der Alzheimer'schen Erkrankung, sowie von Depressionen.

EP 0 659 434 A1

Die Erfindung betrifft die Verwendung von Phosphor- und Phosphonsäureestern zur Behandlung sowie Prävention von Hirnleistungsstörungen und Depressionen, pharmazeutische Mittel und deren Herstellung.

Aus DE 2 642 981, DE 2 209 554, EP 305 840 und EP-279 259 sind Phosphor- und Phosphonsäureester bekannt, die zur Bekämpfung von Insekten eingesetzt werden.

Es wurde nun gefunden, daß Phosphor- und Phosphonsäureester der allgemeinen Formel (I)

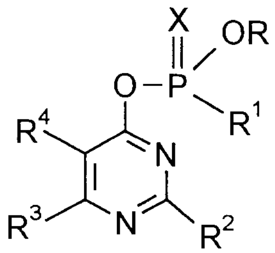

(I)

in welcher

R - für geradkettiges oder verzweigtes Alkyl mit bis zu 6 Kohlenstoffatomen steht,

$R^1$ - für geradkettiges oder verzweigtes Alkyl oder Alkoxy mit bis zu 6 Kohlenstoffatomen steht,

$R^2$ - für Wasserstoff oder
für geradkettiges oder verzweigtes Alkyl, Alkoxy oder Alkylthio mit bis zu 6 Kohlenstoffatomen oder für Dialkylamino mit bis zu 4 Kohlenstoffatomen je Alkylgruppe steht, oder
für Cycloalkyl mit 3 bis 7 Kohlenstoffatomen steht,

$R^3$ - für geradkettiges oder verzweigtes Alkoxy oder Alkyl mit bis zu 6 Kohlenstoffatomen steht, das durch Alkoxy mit bis zu 4 Kohlenstoffatomen substituiert sein kann, oder
- für Wasserstoff steht,

$R^4$ - für Halogen, Wasserstoff oder geradkettiges oder verzweigtes Alkyl oder Alkoxy mit bis zu 4 Kohlenstoffatomen steht,

und

X - Sauerstoff oder Schwefel bedeutet,

und deren physiologisch verträgliche Salze sowie deren Isomere zur Behandlung und Prävention von Hirnleistungsstörungen und Depressionen, eingesetzt werden können.

Aufgrund der Versuchsergebnisse sind die Verbindungen der Formel (I) geeignet zur Behandlung und Prävention von Hirnleistungsstörungen, insbesondere zur Behandlung von seniler und präseniler Demenz, Demenz des Alzheimer-Typs, AIDS-bezogener Demenz, kognitiven Defiziten bei Parkinson oder Hirnleistungsstörungen sowie in Folge von Infarktgeschehen, und zur Behandlung von Depressionen.

Besonders geeignet zur Behandlung und Prävention von Hirnleistungsstörungen und Depressionen sind die Verbindungen der Formel (I),

in welcher

R für geradkettiges oder verzweigtes Alkyl mit bis zu 4 Kohlenstoffatomen steht,

$R^1$ für geradkettiges oder verzweigtes Alkyl oder Alkoxy mit bis zu 4 Kohlenstoffatomen steht,

$R^2$ für Wasserstoff oder
für geradkettiges oder verzweigtes Alkyl oder Alkoxy mit bis zu 4 Kohlenstoffatomen oder
für Dialkylamino mit jeweils bis zu 3 Kohlenstoffatomen je Alkylgruppe steht, oder
für Cycloalkyl mit 3 bis 6 Kohlenstoffatomen steht,

$R^3$ für geradkettiges oder verzweigtes Alkyl oder Alkoxy mit bis zu 4 Kohlenstoffatomen steht, oder für Methoxymethyl steht, oder für Wasserstoff steht,

$R^4$ für Wasserstoff oder Methoxy steht,

und

X für Schwefel steht,

und deren Salze.

Besonders bevorzugt werden Verbindungen der Formel (I) verwendet,

in welcher

R für Methyl steht,

$R^1$ für Methyl, Ethyl, Propyl oder Methoxy steht,

$R^2$ für Cyclopropyl, Methyl, Ethyl, Isopropyl, tert.-Butyl, oder für Methoxy oder Ethoxy steht, oder für Dimethylamino steht,

$R^3$ für Methyl, Ethyl, Alkoxy mit bis zu 4 Kohlenstoffatomen oder Methoxymethyl steht,
$R^4$ für Wasserstoff oder Methoxy steht,
und deren Salze.

Darüber hinaus betrifft die vorliegende Erfindung die im folgenden aufgezählten neuen Stoffe:
O-(6-Methoxy-2-tert.-butyl-pyrimidin-4-yl)-O-methyl-thionomethanphosphonsäure-diester,
O-(6-n-Butyloxy-2-tert.-butyl-pyrimidin-4-yl)-O-methyl-thionoethanphosphonsäure-diester,
O-(6-Methyl-2-tert.-butyl-pyrimidin-4-yl)-O-methyl-thionomethanphosphonsäure-diester,
O-(6-Methoxy-2-tert.-butyl-pyrimidin-4-yl)-O-methyl-thionopropanphosphonsäure-diester,
O-(6-Methyl-2-isopropyl-pyrimidin-4-yl)-O-methyl-thionomethanphosphonsäure-diester,
O-(6-Ethoxy-2-tert.-butyl-pyrimidin-4-yl)-O-methyl-thionomethanphosphonsäure-diester,
O-(6-Propoxy-2-tert.-butyl-pyrimidin-4-yl)-O-methyl-thionoethanphosphonsäure-diester,
O-(6-Propoxy-2-tert.-butyl-pyrimidin-4-yl)-O-methyl-thionomethanphosphonsäure-diester,
O-(6-Butoxy-2-tert.-butyl-pyrimidin-4-yl)-O-methyl-thionomethanphosphonsäure-diester,
O-(6-Methyl-2-cyclopropyl-pyrimidin-4-yl)-0,0-dimethyl-thionophosphorsäure-triester,
O-(6-Methoxy-2-tert.-butyl-pyrimidin-4-yl)-0,0-dimethyl-thionophosphorsäure-triester,
O-(6-Ethoxy-2-tert.-butyl-pyrimidin-4-yl)-0,0-dimethyl-thionophosphorsäure-triester,
O-(6-Propoxy-2-tert.-butyl-pyrimidin-4-yl)-0,0-dimethyl-thionophosphorsäure-triester,
O-(6-Butoxy-2-tert.-butyl-pyrimidin-4-yl)-0,0-dimethyl-thionophosphorsäure-triester,
O-(6-Propoxy-2-cyclopropyl-pyrimidin-4-yl)-0,0-dimethyl-thionophosphorsäure-triester,
O-(5-Methoxy-2-tert.-butyl-pyrimidin-4-yl)-0,0-dimethyl-thionophosphorsäure-triester.

Die erfindungsgemäß verwendeten neuen und bekannten Verbindungen der Formel (I), werden hergestellt indem man

[A] Phosphonsäurehalogenide der Formel (II)

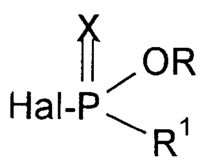

(II)

in welcher
R, $R_1$ und X die angegebene Bedeutung haben und
Hal für Halogen bevorzugt Chlor steht,
mit 4-Hydroxy-pyrimidinen der Formel III

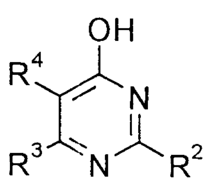

(III)

in welcher
$R^2$, $R^3$ und $R^4$ die angegebene Bedeutung haben,
gegebenenfalls in Gegenwart von säurebindenden Mitteln oder gegebenenfalls in Form der Alkali-, Erdalkali- oder Ammoniumsalzen und gegebenenfalls in Gegenwart von Lösemitteln umsetzt.

Die Ausgangsstoffe der Formel II und III sind bekannt oder nach bekannten Methoden herstellbar.

Das Verfahren zur Herstellung der Verbindungen der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petroether, Benzin Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Di- butylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z.B. Acetonitril und Propionitril, Amide wie z.B. Dietmethylformamid, Dimethylacetamid und N-Methylpyrrolidon sowie Dimethylsulfoxid, Tetramethylensulfon und Hexamethylphosphorsäuretriamid.

Als Säureakzeptoren können üblicherweise für derartige Umsetzungen verwendbaren Säurebindemittel eingesetzt werden. Vorzugsweise in Frage kommen Alkalimetall- und Erdalkalimetallhydride, wie Lithium-, Natrium-, Kalium- und Calcium-hydrid, Alkalimetall- und Erdalkalimetallhydroxide, wie Lithium-, Natrium-, Kalium- und Calcium-hydroxid, Alkalimetall- und Erdalkalimetall-carbonate und -hydrogencarbonate, wie Natrium- und Kaliumcarbonat oder -hydrogencarbonat sowie Calciumcarbonat, Alkalimetallacetate, wie Natrium- und Kalium-acetat, Alkalimetallalkoholate, wie Natrium- und Kalium-tert.-butylat, ferner basische Stickstoffverbindungen, wie Trimethylamin, Triethylamin, Tripropylamin, Tributylamin, Diisobutylamin, Dicyclohexylamin, Ethyldiisopropylamin, Ethyldicyclohexylamin, N,N-Dimethylbenzylamin, N,N-Dimethyl-anilin, Pyridin, 2-Methyl-, 3-Methyl-, 4-Methyl-, 2,4-Dimethyl-, 2,6-Diethyl-, 2-Ethyl-, 4-Ethyl- und 5-Ethyl-2-methylpyridin, 1,5-Diazabicyclo[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können in einem größeren Bereich variiert werden. Im allgemeinen arbeitet man bei Temperaturen zwischen 0 °C und 100 °C, vorzugsweise bei Temperaturen zwischen 10 °C und 80 °C.

Das Verfahren wird im allgemeinen unter Normaldruck durchgeführt. Es ist jedoch auch möglich, unter erhöhtem oder vermindertem Druck zu arbeiten.

Zur Durchführung des Verfahrens werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktion wird im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt jeweils nach üblichen Methoden (vgl. die Herstellungsbeispiele).

Die Verbindungen fallen in den meisten Fällen in Form von Ölen an, die sich zum Teil nicht unzersetzt destillieren lassen, jedoch durch sogenanntes "Andestillieren", d.h. durch längeres Erhitzen unter vermindertem Druck auf mäßig erhöhte Temperaturen von den letzten flüchtigen Anteilen befreit und auf diese Weise gereinigt werden. Zu ihrer Charakterisierung dient der Brechungsindex.

Die Wirksamkeit der Verbindungen der Formel (I) bei der Behandlung und Prävention Hirnleistungsstörungen wird mit Hilfe eines Tiermodells belegt, welches auf dem räumlichen Gedächtnis beruht. In diesem ursprünglich von Morris beschriebenen Test (R.G.M. Morris, J. Neurosci. Methods 11:47-60, 1984) müssen Ratten lernen, eine für sie unsichtbare Plattform als einzige Auswegmöglichkeit aus einem Wasserpool zu lokalisieren. Dazu bekommen die Tiere (junge, adulte, männliche Ratten, ca. 3 Monate alt) täglich 4 Trainingsläufe über einen Zeitraum von 3 Tagen. Gemessen wird die Latenzzeit für das Auffinden der Plattform und die zurückgelegte Strecke. Während des fortschreitenden Trainings verkürzt sich sowohl die Latenzzeit als auch die Schwimmstrecke durch die Aneignung einer Strategie zur räumlichen Lokalisation des Ziels. Die kognitiv verbessernde Wirkung von Prüfsubstanzen manifestiert sich in einer Beschleunigung der für den Lernprozess benötigten Zeit, d.h. die Lernkurve wird steiler. Testsubstanzen werden täglich einmal (60 min vor dem ersten Trainingslauf) appliziert. Kontrolltiere erhalten eine entsprechende Menge des Vehikels.

Am Ende der Trainingsphase, d.h. nach Ablauf des 12. Trainingslaufs wird die Plattform aus dem Wasser genommen und in einem weiteren Schwimmversuch geprüft, ob die Versuchstiere in einem definierten engen Umkreis der Trainingsposition der Plattform nach dieser suchen (Retentionstest).

Die Testergebnisse zeigen, daß die beschriebenen Verbindungen das Lernverhalten der Tiere wie auch die Retention des Gelernten positiv beeinflussen. Die beschriebenen Ester können daher sowohl zur therapeutischen als auch zur präventiven Behandlung von kognitiven Störungen allgemein, insbesondere von Demenzen des Alzheimer-Typs, verwendet werden.

Die Wirksamkeit der Verbindungen der Formel (I) bei der Behandlung und Prävention von Depressionen wird mit Hilfe des "Rat Forced Swimming Tests" belegt. Dieses Verhaltensmodell wurde erstmalig von Porsolt et al. (R.D. Porsolt, M. Le Pichon, M. Jalfre, Nature 266:730-732, 1977) beschrieben und ist heute ein allgemein anerkanntes in-vivo Screeningmodell für die Auffindung neuer Antidepressiva. Er beruht auf der Beobachtung, daß Ratten in einer ausweglosen Situation in einer unbeweglichen Stellung verharren ("behavioural despair"). In einem Vorversuch werden junge, adulte Ratten (3-4 Monate alt) für 20 min einzeln in Glaszylinder (Höhe 40 cm, Durchmesser 20 cm) gesetzt, welche bis zu einer Höhe von 15 cm mit Wasser gefüllt sind. 24 h nach diesem Vortest werden die Tiere wiederum in die Zylinder überführt, und die Dauer der Immobilität über einen Zeitraum von 5 min gemessen. Die beschriebenen Ester werden im

Zeitintervall zwischen den beiden Schwimmversuchen appliziert. Kontrollen erhalten das Vehikel.

Analog zu in der Literatur beschriebenen, klinisch aktiven Antidepressiva verkürzen die Verbindungen der Formel (I) die Immobilitätsdauer und führen zu einer Verhaltensaktivierung. Aufgrund dieser Ergebnisse sind die beschriebenen Verbindungen auch zur Behandlung affektiver Störungen, insbesondere der Depression, geeignet.

Testergebnisse:

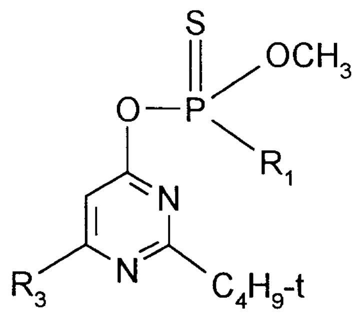

| $R_1$ | $R_3$ | $ED_{50}$ oral Rat Forced Swim Test | Wirkdosen oral Morris Maze |
|---|---|---|---|
| $-CH_3$ | $-OCH_3$ | 0,11 mg/kg | 0,3-1 mg/kg |
| $-C_2H_5$ | $-OCH_3$ | 3,0 mg/kg | 30 mg/kg |
| $-CH_3$ | $-CH_3$ | 0,24 mg/kg | 0,1-0,3 mg/kg |
| $-CH_3$ | $-OC_4H_9$-n | 0,17 mg/kg | 0,1 mg/kg |
| $-CH_3$ | $-OC_3H_7$-n | 0,015 mg/kg | not tested |
| $-C_2H_5$ | $-OC_3H_7$-n | 0,014 mg/kg | " |
| $-OCH_3$ | $-OCH_3$ | 0,55 mg/kg | 1-3 mg/kg |
| $-OCH_3$ | $-OC_2H_5$ | 2,3 mg/kg | not tested |
| $-OCH_3$ | $-OC_3H_7$ | 1,3 mg/kg | 1-3 mg/kg |

Die Erfindung umfaßt ebenfalls pharmazeutische Mittel, die die genannten Verbindungen in einer wirksamen Menge enthalten, deren Herstellung und Verwendung zur Behandlung und Prävention der vorstehend genannten Krankheiten.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen unter Verwendung inerter, nichttoxischer, pharmazeutisch geeigneter Trägerstoffe oder Lösemittel.

Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungspielraum zu erreichen.

Die Formulierungen werden beispielsweise hergestellt durch Verstrecken der Wirkstoffe mit Lösemitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösemittel oder Hilfslösemittel verwendet werden können.

Die Applikation erfolgt in üblicher Weise, vorzugsweise oral oder parenteral, insbesondere perlingual oder intravenös. Auch eine transdermale Applikation ist möglich z.B. durch Pflaster.

Für den Fall der parenteralen Anwendung können Lösungen des Wirkstoffs unter Verwendung geeigneter Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1

bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

**Herstellungsbeispiele:**

**Beispiel 1:**

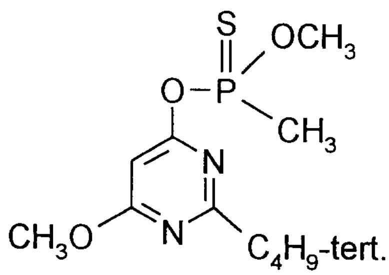

7,3 g (0,05 Mol) Thionomethanphosphonsäurechlorid-methylester werden bei 20 °C unter Rühren zu einer Mischung aus 9,1 g (0,05 Mol) 4-Hydroxy-6-methoxy-2-tert.-butyl-pyrimidin (Herstellung s. EP-279 259), 10,3 g (0,075 Mol) Kaliumcarbonat und 80 ml Acetonitril gegeben. Man rührt das Reaktionsgemisch 15 Stunden bei 20 °C nach und destilliert dann das Lösungsmittel im Vakuum ab. Der Rückstand wird in 100 ml Methyl-tert.-butylether aufgenommen und zweimal mit je 50 ml Wasser gewaschen, mit Natriumsulfat getrocknet und filtriert. Vom Filtrat wird das Lösungsmittel bei ca. 50 °C in Vakuum abdestilliert.

Man erhält 7,7 g (53 % der Theorie) O-(6-Methoxy-2-tert.-butyl-pyrimidin-4-yl)-O-methyl-thionomethanphosphonsäure-diester als öligen Rückstand vom Brechungsindex $n_D^{23}$ : 1,5121.

**Beispiel 2:**

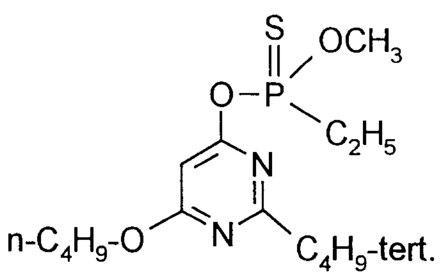

6,85 g (0,05 Mol) n-Butylbromid werden bei 20 °C unter Rühren zu einer Mischung aus 11,6 g (0,04 Mol) O-(6-Hydroxy-2-tert.-butyl-pyrimidin-4-yl)-O-methyl-thionoethanphosphonsäure-diester (Herstellung s. EP-279 259), 6,9 g (0,05 Mol) Kaliumcarbonat und 80 ml Acetonitril gegeben. Man rührt das Reaktionsgemisch 15 Stunden bei 20 °C nach und destilliert dann das Lösungsmittel im Vakuum ab. Der Rückstand wird in 100 ml Methyl-tert.-butylether aufgenommen und einmal mit 50 ml 5-prozentiger Natronlauge und einmal mit 50 ml Wasser gewaschen, mit Natriumsulfat getrocknet und filtriert. Vom Filtrat wird das Lösungsmittel bei ca. 50 °C im Vakuum destilliert.

Man erhält 6,55 g (47 % d. Theorie) O-(6-n-Butyloxy-2-tert.-butyl-pyrimidin-4-yl)-O-methyl-thionoethanphosphonsäure-diester als öligen Rückstand vom Brechungsindex $n_D^{25}$ : 1,4961.

Analog Beispiel 1 erhält man die folgenden Verbindungen der Formel

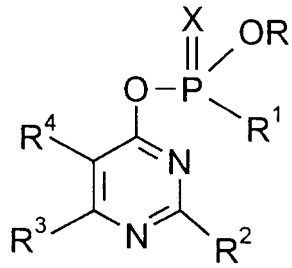

| Bsp.-Nr. | R | $R^1$ | $R^2$ | $R^3$ | $R^4$ | X | Brechungsindex ($n_D$/ ° C) |
|---|---|---|---|---|---|---|---|
| 3 | $CH_3$ | $CH_3$ | t.-$C_4H_9$ | -$CH_3$ | H | S | 1,5092/27 |
| 4 | $CH_3$ | n-$C_3H_7$ | t.-$C_4H_9$ | -$OCH_3$ | H | S | 1,5042/23 |
| 5 | $CH_3$ | $CH_3$ | i-$C_3H_7$ | -$CH_3$ | H | S | |
| 6 | $CH_3$ | $CH_3$ | t.-$C_4H_9$ | -$OC_2H_5$ | H | S | 1,5058/20 |
| 7 | $CH_3$ | $C_2H_5$ | t.-$C_4H_9$ | -$OC_3H_7$(n) | H | S | 1,5028/20 |
| 8 | $CH_3$ | $CH_3$ | t.-$C_4H_9$ | -$OC_3H_7$(n) | H | S | 1,5032/20 |
| 9 | $CH_3$ | $CH_3$ | t.-$C_4H_9$ | -$OC_4H_9$(n) | H | S | 1,4987/23 |

**Herstellungsbeispiele:**

**Beispiel 10:**

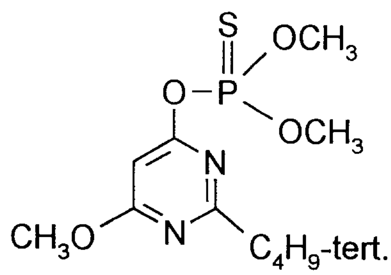

16 g (0,1 Mol) Thionophosphorsäurechlorid-dimethylester werden bei 20 ° C unter Rühren zu einer Mischung aus 18,2 g (01 Mol) 4-Hydroxy-6-methoxy-2-tert.-butyl-pyrimidin, 20,8 g (0,15 Mol) Kaliumcarbonat und 80 ml Acetonitril gegeben. Man rührt das Reaktionsgemisch 15 Stunden bei 20 ° C nach und destilliert dann das Lösungsmittel im Vakuum ab. Der Rückstand wird in 100 ml Toluol aufgenommen und zweimal mit je 100 ml Wasser gewaschen, mit Natriumsulfat getrocknet und filtriert. Vom Filtrat wird das Lösungsmittel bei ca. 50 ° C in Vakuum abdestilliert. Den Rückstand befreit man bei 70 ° C im Hochvakuum von flüchtigen Anteilen.

Man erhält 27,6 g (90 % der Theorie) O-(6-Methoxy-2-tert.-butyl-pyrimidin-4-yl)-0,0-dimethyl-thionophosphorsäure-triester als öligen Rückstand vom Brechungsindex $n_D^{21}$ : 1,5069.

Analog Beispiel 1 werden die folgenden Verbindungen der Formel erhalten

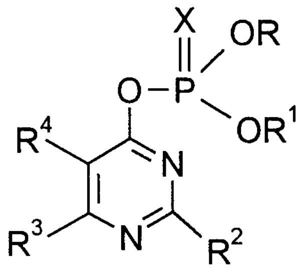

| Bsp.-Nr. | R | $R^1$ | $R^2$ | $R^3$ | $R^4$ | X | Brechungsindex ($^nD$/°C) |
|---|---|---|---|---|---|---|---|
| 11 | $CH_3$ | $CH_3$ | 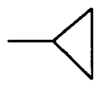 | $CH_3$ | H | S | |
| 12 | $CH_3$ | $CH_3$ | $C_4H_9$-tert. | $C_2H_5O$ | H | S | 1,4970/20 |
| 13 | $CH_3$ | $CH_3$ | $C_4H_9$-tert. | n-$C_3H_7O$ | H | S | 1,495 6/20 |
| 14 | $CH_3$ | $CH_3$ | $C_4H_9$-tert. | n-$C_4H_9O$ | H | S | |
| 15 | $CH_3$ | $CH_3$ | 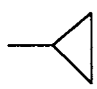 | n-$C_3H_7O$ | H | S | |
| 16 | $CH_3$ | $CH_3$ | $C_4H_9$-tert. | H | $CH_3O$ | S | |

## Patentansprüche

**1.** Verwendung von Phosphor- und Phosphonsäureester der allgemeinen Formel (I)

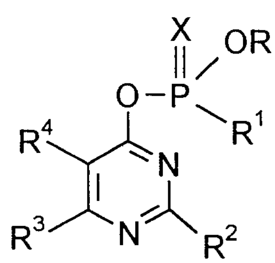

(I)

in welcher

- R - für geradkettiges oder verzweigtes Alkyl mit bis zu 6 Kohlenstoffatomen steht,
- $R^1$ - für geradkettiges oder verzweigtes Alkyl oder Alkoxy mit bis zu 6 Kohlenstoffatomen steht,
- $R^2$ - für Wasserstoff oder für geradkettiges oder verzweigtes Alkyl, Alkoxy oder Alkylthio mit bis zu 6 Kohlenstoffatomen oder für Dialkylamino mit bis zu 4 Kohlenstoffatomen je Alkylgruppe steht, oder für Cycloalkyl mit 3 bis 7 Kohlenstoffatomen steht,

$R^3$ - für geradkettiges oder verzweigtes Alkoxy oder Alkyl mit bis zu 6 Kohlenstoffatomen steht, das durch Alkoxy mit bis zu 4 Kohlenstoffatomen substituiert sein kann, oder für Wasserstoff steht,

$R^4$ - für Halogen, Wasserstoff oder geradkettiges oder verzweigtes Alkyl oder Alkoxy mit bis zu 4 Kohlenstoffatomen steht,

und

X - Sauerstoff oder Schwefel bedeutet,

und deren physiologisch verträglichen Salze sowie deren Isomere zur Behandlung und Prävention von Hirnleistungsstörungen und Depressionen.

**2.** Verwendung nach Anspruch 1, wobei in der allgemeinen Formel

R für geradkettiges oder verzweigtes Alkyl mit bis zu 4 Kohlenstoffatomen steht,

$R^1$ für geradkettiges oder verzweigtes Alkyl oder Alkoxy mit bis zu 4 Kohlenstoffatomen steht,

$R^2$ für Wasserstoff oder für geradkettiges oder verzweigtes Alkyl oder Alkoxy mit bis zu 4 Kohlenstoffatomen oder für Dialkylamino mit jeweils bis zu 3 Kohlenstoffatomen je Alkylgruppe steht, oder für Cycloalkyl mit 3 bis 6 Kohlenstoffatomen steht,

$R^3$ für geradkettiges oder verzweigtes Alkyl oder Alkoxy mit bis zu 4 Kohlenstoffatomen steht, oder für Methoxymethyl steht, oder für Wasserstoff steht

$R^4$ für Wasserstoff oder Methoxy steht,

und

X für Schwefel steht,

und deren Salze.

**3.** Verwendung nach Anspruch 1, wobei in der allgemeinen Formel

R für Methyl steht,

$R^1$ für Methyl, Ethyl, Propyl oder Methoxy steht,

$R^2$ für Cyclopropyl, Methyl, Ethyl, Isopropyl, tert.-Butyl, oder für Methoxy oder Ethoxy steht, oder für Dimethylamino steht,

$R^3$ für Methyl, Ethyl, Alkoxy mit bis zu 4 Kohlenstoffatomen oder Methoxymethyl steht,

$R^4$ für Wasserstoff oder Methoxy steht,

und deren Salze.

**4.** Phosphor- und Phosphonsäureester der Reihe
O-(6-Methoxy-2-tert.-butyl-pyrimidin-4-yl)-O-methylthionomethanphosphonsäure-diester,
O-(6-n-Butyloxy-2-tert.-butyl-pyrimidin-4-yl)-O-methylthionoethanphosphonsäure-diester,
O-(6-Methyl-2-tert.-butyl-pyrimidin-4-yl)-O-methyl-thionomethanphosphonsäure-diester,
O-(6-Methoxy-2-tert.-butyl-pyrimidin-4-yl)-O-methylthionopropanphosphonsäure-diester,
O-(6-Methyl-2-isopropyl-pyrimidin-4-yl)-O-methylthionomethanphosphonsäure-diester,
O-(6-Ethoxy-2-tert.-butyl-pyrimidin-4-yl)-O-methyl-thionomethanphosphonsäure-diester,
O-(6-Propoxy-2-tert.-butyl-pyrimidin-4-yl)-O-methyl-thionoethanphosphonsäure-diester,
O-(6-Propoxy-2-tert.-butyl-pyrimidin-4-yl)-O-methylthionomethanphosphonsaure-diester,
O-(6-Butoxy-2-tert.-butyl-pyrimidin-4-yl)-O-methyl-thionomethanphosphonsäure-diester.
O-(6-Methyl-2-cyclopropyl-pyrimidin-4-yl)-0,0-dimethyl-thionophosphorsäure-triester,
O-(6-Methoxy-2-tert.-butyl-pyrimidin-4-yl)-0,0-dimethyl-thionophosphorsäure-triester,
O-(6-Ethoxy-2-tert.-butyl-pyrimidin-4-yl)-0,0-dimethyl-thionophosphorsäure-triester,
O-(6-Propoxy-2-tert.-butyl-pyrimidin-4-yl)-0,0-dimethyl-thionophosphorsäure-triester,
O-(6-Butoxy-2-tert.-butyl-pyrimidin-4-yl)-0,0-dimethyl-thionophosphorsäure-triester,
O-(6-Propoxy-2-cyclopropyl-pyrimidin-4-yl)-0,0-dimethyl-thionophosphorsäure-triester,
O-(5-Methoxy-2-tert.-butyl-pyrimidin-4-yl)-0,0-dimethyl-thionophosphorsäure-triester.

**5.** Arzneimittel enthaltend die Phosphor- und Phosphonsäureester nach Anspruch 4.

**6.** Phosphor- und Phosphonsäureester nach Anspruch 4 zur Behandlung von Krankheiten.

**7.** Verfahren zur Herstellung von Phosphor- und Phosphonsäureester nach Anspruch 4 dadurch gekennzeichnet, daß man die jeweiligen Phosphor- bzw. Phosphonsäurehalogenide mit den entsprechenden 4-Hydroxy-pyrimidinen gegebenenfalls in Gegenwart von säurebindenden Mitteln oder gegebenenfalls in

Form von Alkali-, Erdalkali- oder Ammoniumsalzen und gegebenenfalls in Gegenwart eines Lösemittels umsetzt.

Europäisches Patentamt

# EUROPÄISCHER RECHERCHENBERICHT

Nummer der Anmeldung
EP 94 11 9398

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| A | WO-A-90 06122 (THE BOARD OF TRUSTEES OF SOUTHERN ILLINOIS UNIVERSITY)<br>* das ganze Dokument *<br>--- | 1-7 | A61K31/675<br>C07F9/6512 |
| D,A | FR-A-2 365 578 (BAYER AG)<br>* das ganze Dokument *<br>--- | 1-7 | |
| D,A | FR-A-2 128 558 (SANDOZ SA)<br>* das ganze Dokument *<br>--- | 1-7 | |
| D,A | EP-A-0 305 840 (BAYER AG)<br>* das ganze Dokument *<br>--- | 1-7 | |
| D,A | EP-A-0 279 259 (BAYER AG)<br>* das ganze Dokument *<br>--- | 1-7 | |
| A | US-A-4 012 506 (DAVID E. BALKE)<br>* das ganze Dokument *<br>----- | 1-7 | |
| | | | RECHERCHIERTE SACHGEBIETE (Int.Cl.6) |
| | | | A61K<br>C07F |

Der vorliegende Recherchenbericht wurde für alle Patentansprüche erstellt

| Recherchenort | Abschlußdatum der Recherche | Prüfer |
|---|---|---|
| DEN HAAG | 10. März 1995 | Beslier, L |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)